# FASCICULE DE BREVET EUROPEEN

(11) **EP 3 355 906 B1**
(45) Date de publication et mention de la délivrance du brevet: **02.09.2020**
(21) Numéro de dépôt: 16788156.4
(22) Date de dépôt: 29.09.2016
(51) Int. Cl.: A61K 38/13, A61K 31/728, A61K 9/00, A61P 27/02

(54) **PROCEDE DE PREPARATION D'UN COLLYRE DE CICLOSPORINE A**
VERFAHREN ZUR HERSTELLUNG VON AUGENTROPFEN AUS CYCLOSPORIN A
METHOD FOR PREPARING EYE DROPS OF CYCLOSPORIN A

(30) Priorité: 02.10.2015 FR 1559367
(43) Date de publication de la demande: 08.08.2018
(73) Titulaire: CHU Clermont-Ferrand, 63003 Clermont-Ferrand cedex 1 (FR)
(72) Inventeur: CHENNELL, Philip, 63170 Aubiere (FR); CHIAMBARETTA, Frédéric, 63000 Clermont-Ferrand (FR); DELABORDE, Lucie, 63000 Clermont-Ferrand (FR); JOUANNET, Mireille, 63000 Clermont-Ferrand (FR); SAUTOU, Valérie, 63000 Clermont-Ferrand (FR)
(74) Mandataire: Dennemeyer & Associates S.A.
(86) Numéro de dépôt international: PCT/FR2016/052492
(87) Numéro de publication internationale: WO 2017/055758

(56) Documents cités:
- EP-A1- 2 845 602
- WO-A1-95/31211
- US-A1- 2007 087 962
- US-A1- 2008 299 206
- Allergan: "HIGHLIGHTS OF PRESCRIBING INFORMATION These highlights do not include all the information needed to use RESTASIS 0.05% safely and effectively. See full prescribing information for RESTASIS FULL PRESCRIBING INFORMATION: CONTENTS* 1 INDICATIONS AND USAGE 2 DOSAGE AND ADMINISTRATION 3 DOSAGE FORMS AN", , 1 juin 2013 (2013-06-01), pages 1-6, XP055284055, Extrait de l'Internet: URL:http://www.allergan.com/assets/pdf/res tasis_pi.pdf [extrait le 2016-06-28]
- F Chast ET AL: "Préparation d'un collyre de ciclosporine à 2 % Cyclosporine 2% eye drops preparation", J Fr. Ophtalmol. J. Fr. Ophtalmol, 1 janvier 2004 (2004-01-01), pages 567-576, XP055283877, Extrait de l'Internet: URL:http://www.em-consulte.com/showarticle file/112835/index.pdf [extrait le 2016-06-27]

## Description

La présente invention concerne un procédé de préparation d'un collyre de ciclosporine A.

Les ciclosporines sont des oligopeptides cycliques ayant des activités immunosuppressives et anti-inflammatoires de la famille des anticalcineurines. Parmi les ciclosporines, la ciclosporine A est une molécule entrant dans la formulation d'un certain nombre de médicaments, pour le traitement de diverses pathologies inflammatoires, que ce soit par voie générale ou locale. En ophtalmologie, des collyres de ciclosporine A de 0,5 à 20 mg/ml sont préconisés pour le traitement de pathologies inflammatoires chroniques de l'œil tel que, par exemple, la kératoconjonctivite vernale, le syndrome sec résistant au traitement de 1ère intention, la prévention ou le traitement de rejet de greffe de cornée.

Les ciclosporines étant lipophiles, elles sont essentiellement employées dans des formulations à base d'huile. Pour une administration locale par voie oculaire, les huiles couramment utilisées comme véhicule du principe actif sont, à titre d'exemples non limitatifs, les huiles d'olive, d'arachide, de ricin, de ricin polyoxyéthylénée ou des huiles minérales. Il s'avère que les formulations de ciclosporine à base d'huile présentent une faible tolérance oculaire, ce qui induit assez fréquemment des effets indésirables tels que des irritations oculaires, des sensations de brûlure à l'instillation, des larmoiements ou encore des altérations de la qualité visuelle.

F. Chast et Al, J Fr. Ophtalmol. (2004-01-01), pages 567-576, XP055283877 décrit la préparation d'un collyre de ciclosporine à 2% avec de l'huile de ricin et en présence d'éthanol. Le collyre obtenu est une solution purement huileuse. Ce document ne prévoit pas l'ajout d'agent lubrifiant et/ou cicatrisant. Il est connu de WO-A-9531211 de diminuer la quantité d'huile et de former une émulsion avec de l'eau. Il est connu de WO-A-2006/050838 une émulsion huile /eau à usage ophtalmique comprenant des particules de colloïdes enrobées d'un film interfacial. Une telle solution est délivrable sous forme de patch, implant ou insert mais elle n'est pas adaptée à un usage comme collyre. WO-A-2005/032577 décrit une formulation ophtalmique pour un collyre destiné particulièrement au traitement de l'œil sec, comportant de l'eau, un composé hydrophobe, une ciclosporine A à une concentration de moins de 1 mg/ml soit moins de 0,1% en poids total. Le composé hydrophobe est choisi parmi des huiles végétales, animales, minérales ou synthétiques ainsi que parmi des mélanges de ces huiles. D'autres composants tels des stabilisants ou des tensioactifs sont également utilisés. Dans un mode de réalisation, la formulation ne contient pas de conservateur. Elle comprend également un composé polyanionique de nature tensioactive ou employé en tant que lubrifiant et cicatrisant tel que, entres autres, de l'acide hyaluronique. Des conservateurs sont, si besoin, incorporés à la formulation.

Or il s'avère qu'il peut être nécessaire d'avoir une concentration en ciclosporine plus élevée, à savoir supérieure, ou au moins égale, à 1 mg/ml soit 0,1% en poids total, voir comprise, par exemple, entre 5 mg/ml et 25 mg/ml.

De telles concentrations sont, par exemple, nécessaires pour le traitement des kératoconjonctivites vernales, pour prévenir et/ou traiter les rejets de greffes de cornée.

Or, il s'avère que de telles préparations ne sont pas actuellement disponibles sur le marché en tant que médicament bénéficiant d'une autorisation de mise sur le marché (AMM) ou d'une autorisation temporaire d'utilisation couvrant l'ensemble des besoins, notamment pour la population pédiatrique. De ce fait, de telles préparations doivent être préparées à façon en milieu hospitalier, par les Pharmacies à Usage Intérieur ou PUI, à savoir les services de pharmacie dédiés au seul organisme hospitalier dont ils dépendent. De ce fait, les formulations et/ou les conditions de préparation ne sont pas homogènes entre les différents établissements, tant en France qu'au niveau Européen. Cela induit de grandes différences dans la tolérance oculaire, les conditions et/ou les durées de conservation de telles préparations. De plus il est fréquent que de telles préparations comprennent, à des concentrations plus ou moins importantes, de l'éthanol, des conservateurs ou soient entièrement huileuses, ce qui pose, par exemple, des problèmes de tolérance à l'instillation dans l'oeil et, de façon chronique, sur la surface cornéenne.

L'invention vise plus particulièrement à proposer une autre méthode de préparation de collyre permettant d'obtenir de plus fortes concentrations en ciclosporine A, typiquement supérieures à 1 mg/ml, et cela sans nécessiter de conservateur, avec une concentration minimale en éthanol, et en offrant une tolérance oculaire optimisée.

A cet effet, l'invention a pour objet un procédé de préparation d'un collyre de ciclosporine A, dont la concentration dans le collyre est comprise entre 1mg/ml et 40 mg/ml, sous forme d'une émulsion, d'une microémulsion ou d'une solution micellaire lipophile/hydrophile dont la phase lipophile, donc huileuse, contient la ciclosporine A, caractérisé en ce qu'il comprend au moins des étapes consistant à :
a) éliminer au moins 99% en masse de l'éthanol contenu dans la solution huileuse de ciclosporine A, sous un flux d'azote sous une pression de 0,5 bar, à une température comprise entre 2°C et 45°C et pendant une durée comprise entre 0,25h et 24h,
b) diluer en solution aqueuse le concentré obtenu à l'étape a),
c) réaliser une émulsion, une microémulsion ou une solution micellaire dont la phase aqueuse représente au moins 75% de l'émulsion, microémulsion ou solution micellaire finale, par mélange sous agitation de la solution huileuse concentrée obtenue à l'étape a) avec la solution aqueuse diluée obtenue à l'étape b),
d) conditionner stérilement le collyre obtenu dans des conditionnements adaptés à un usage ophtalmique.

Selon des aspects avantageux mais non obligatoires de l'invention, un tel procédé peut comprendre une ou plusieurs des caractéristiques suivantes:
- le collyre est préparé sans ajout de conservateur antimicrobien.
- Lors de l'étape b), on incorpore à la solution aqueuse une solution d'agent lubrifiant et/ou cicatrisant.
- L'agent lubrifiant et/ou cicatrisant est choisi parmi l'acide hyaluronique et ses sels ou la povidone.
- La solution d'agent lubrifiant et/ou cicatrisant est reconstituée à partir de poudre ou prête à l'emploi.
- Lors de l'étape d), le collyre est soumis à une stérilisation finale préalablement à son conditionnement.
- La solution huileuse de ciclosporine A est une solution de ciclosporine A dans de l'huile hydro-dispersive et émulsifiante.
- La solution huileuse de ciclosporine A est une solution de ciclosporine A dans de l'huile de ricin polyoxyéthylénée.
- La concentration en cyclosporine A dans le collyre obtenu à l'issue de l'étape d) est comprise entre 5 mg/ml et 25 mg/ml.
- Le collyre obtenu à l'issue de l'étape d) est adapté à un usage humain ou vétérinaire.

L'invention sera mieux comprise et d'autres avantages de celle-ci apparaitront plus clairement à la lecture de la description de plusieurs modes de réalisation de l'invention, donnée à titre d'exemple non limitatif.

Il est rappelé ici qu'un tel procédé est avantageusement mais non exclusivement destiné à une mise en œuvre dans un cadre hospitalier.

Dans de nombreuses maladies inflammatoires chroniques de l'œil, l'utilisation de la ciclosporine en application locale en alternative aux corticoïdes est considérée, de manière connue, comme intéressante. De telles préparations sont effectuées par les Pharmacies à Usage Intérieur ou PUI des établissements de santé, en respectant la réglementation en vigueur, à savoir les Bonnes Pratiques de Préparation entre autres, dans la mesure où aucune spécialité pharmaceutique répondant aux besoins n'est disponible ou adapté à l'usage pressenti.

On conçoit qu'un tel procédé puisse également être mis en œuvre dans le cadre d'une production commerciale, dans le respect de la réglementation en vigueur.

Le procédé objet de l'invention est particulièrement, mais non exclusivement, adapté à la préparation de formulations ayant une concentration en ciclosporine A compris entre 1 mg/ml et 40 mg/ml, et préférentiellement entre 5 mg/ml et 25 mg/ml.

Actuellement les préparations réalisées dans les centres hospitaliers sont majoritairement préparées uniquement à base d'huile. En effet, les ciclosporines sont peu solubles dans l'eau. A titre d'exemple la solubilité de la ciclosporine A est de l'ordre de 20 à 30 µg / ml. En d'autres termes, lorsque des concentrations en ciclosporine A supérieures sont nécessaires, typiquement de l'ordre de 300 à 1000 fois plus, donc des concentrations d'environ 10 mg/ml à 20 mg/ml, une solution aqueuse de ciclosporine A n'est pas réalisable, de manière aisée.

Si l'emploi de formulation à base uniquement d'huile ou de mélanges d'huiles pallie à ce problème, cela génère des problèmes de tolérance. Il est connu de l'état de la technique (Yavuz B et al ; An Overview on Dry Eye Treatment: Approaches for Cyclosporin A Delivery; The Scientific World Journal, 2012; 2012:1-11. Lallemand F, et al; Cyclosporine A delivery to the eye: A pharmaceutical challenge; European Journal of Pharmaceutics and Biopharmaceutics, nov 2003;56(3):307-18. H. Nourry et al. 2006, Étude de la cytotoxicité de différents collyres à base de ciclosporine A buvable (Sandimmun®), Journal Français d'Ophtalmologie, mars 2006;29(3):251-7.) que des formulations à base uniquement huileuse induisent des brûlures, picotements et douleurs pouvant perdurer plusieurs heures après l'application du collyre.

Le fait de réaliser une préparation qui associe un composé lipophile et un composé hydrophile, typiquement une émulsion lipophile/hydrophile ou une microémulsion lipophile/hydrophile ou une solution micellaire dont la phase dispersée est lipophile et contient la ciclosporine A, la phase dispersante de cette solution micellaire étant aqueuse, permet d'obtenir un collyre dont la tolérance est améliorée par rapport à une préparation uniquement lipophile, cela tout en conservant une solubilité supérieure, donc une faisabilité supérieure, à celle des préparations purement aqueuses.

Il est fréquent d'incorporer un conservateur afin de pouvoir optimiser la durée de d'utilisation de ces préparations, par exemple après ouverture du conditionnement. Le conservateur, ou un mélange de conservateurs, est, à titre d'exemples non limitatifs, choisi parmi des ammoniums quaternaires, des dérivés mercuriels, des alcools, des sels de chlorohexidrine. Il est connu et admis que les conservateurs sont potentiellement toxiques pour l'endothélium cornéen, en raison d'une part de leur cytotoxicité directe et, d'autre part, de leur effet détergent qui altère le film lacrymal et provoque des sécheresses oculaires. Ces problèmes sont particulièrement présents lorsque l'on utilise des ammoniums quaternaires comme conservateurs. A titre d'exemples non limitatifs de conservateurs on peut citer le chlorure de benzalkonium, le cétrimide, le polysorbate, l'EDTA ou Éthylène Diamine Tétra-Acétate, le chlorobutanol, le perborate de potassium.

Le fait de réaliser une préparation sans conservateur antimicrobien permet d'obtenir un collyre dont la tolérance est améliorée par rapport à un collyre qui en contiendrait.
La phase aqueuse d'une telle préparation permet, de façon avantageuse mais non obligatoire, d'incorporer des additifs, au sens de composés additionnels actifs, à la préparation. De manière préférée, la phase aqueuse permet d'incorporer un composé lubrifiant et/ou cicatrisant. Parmi ceux-ci on peut citer, à titre non limitatif, l'acide hyaluronique/hyaluronate de sodium, la povidone, les carbomères, l'alcool polyvinylique, la polyvidone, les dextrans, les polysaccharides, la famille des carboxy ou méthyl ou propyl cellulose, la glycérine ou glycérol. Il est connu, par exemple par EP-A-1142566, d'améliorer la biodisponibilité et la tolérance d'une émulsion huile eau de ciclosporine en incorporant de l'acide hyaluronique ou un de ses sels. Une telle incorporation permet d'augmenter la viscosité du collyre, favorisant ainsi la bonne répartition du collyre sur la surface oculaire et améliorant son efficacité. Il est également utilisé pour ses propriétés lubrifiante et/ou cicatrisante ce qui améliore la tolérance à l'instillation et la stabilité du film lacrymal.

La présente invention concerne une préparation ophtalmique de type émulsion lipophile/hydrophile ou microémulsion lipophile/hydrophile ou solution micellaire dont la phase dispersée est lipophile et contient la ciclosporine A, la phase dispersante de cette solution micellaire étant aqueuse, à une concentration d'au moins 1 mg/ml, sans conservateur et avec un agent lubrifiant et/ou cicatrisant.
En particulier, à titre d'exemples non limitatifs, la demanderesse a réalisé deux formulations de type émulsion dans eau, sans conservateur, avec deux agents lubrifiants et/ou cicatrisants différents, et cela pour trois concentrations différentes en ciclosporine A. Les exemples suivants sont donnés pour des concentrations de 1, 10 et 20 mg/ml. On conçoit que l'invention permet de réaliser toute concentration en ciclosporine A comprise entre 1 et 40 mg/ml.

### Exemple 1 :

Préparation avec de l'acide hyaluronique comme agent lubrifiant et cicatrisant.
L'acide hyaluronique est soit sous forme acide soit sous forme d'un sel tel qu'un hyaluronate de sodium, un hyaluronate de potassium, un hyaluronate de calcium. Avantageusement, on utilise un mélange d'acide hyaluronique et d'hyaluronate de sodium en solution aqueuse (eau pour préparation injectable ou PPI) et éventuellement du chlorure de sodium, du phosphate mono et disodique ainsi que de l'acide chlorhydrique. Le mélange est prêt à l'emploi ou réalisé extemporanément avec dissolution des différents composés sous agitation.

L'huile utilisée est choisie parmi une huile végétale, animale, minérale ou synthétique, qui possède, de préférence, des propriétés hydro-dispersives, émulsifiantes et assurant la solubilité des composés lipophiles tout en permettant la réalisation de l'émulsion. En variante, il s'agit d'une microémulsion ou d'une solution micellaire. On peut citer, à titre d'exemples, des composés de nature huileuse du type glycérol, polysorbate 80 ou des glycérides de synthèse.

Parmi les glycérides de synthèse, une huile de ricin polyoxyéthylénée est préférentiellement utilisée avec un ratio molaire compris entre 25 et 50 et, préférentiellement, entre 35 et 45.

Le tableau 1 suivant donne les formulations détaillées d'un mode de préparation pour des concentrations en ciclosporine de 1, 10 et 20 mg/ml.

Ici, l'agent lubrifiant et cicatrisant est à base d'acide hyaluronique.

**Tableau 1**

| | Formulation 1 | | |
|---|---|---|---|
| Ciclosporine | 1 mg/ml | 10 mg/ml | 20 mg/ml |
| Huile de ricin polyoxyéthylénée | 13 mg/ml | 130 mg/ml | 260 mg/ml |
| Ethanol | 0-5,56 mg/ml | 0 - 5,56 mg/ml | 0 - 11,12 mg/ml |
| Azote dissout | Traces éventuelles | Traces éventuelles | Traces éventuelles |
| Solution aqueuse (eau PPI) | QSP | QSP | QSP |
| dont : Acide Hyaluronique / hyaluronate de sodium | 1,47 mg/ml | 1,305 mg/ml | 1,125 mg/ml |
| dont : Chlorure de sodium, Phosphate mono et di sodique, acide chlorhydrique | | | |
| % de phase huileuse | 2% | 12,50% | 25% |
| % de phase aqueuse | 98% | 87,50% | 75% |

Une variation de plus ou moins 10% des valeurs de concentration cible indiquées est acceptable.

La concentration en ciclosporine A est mesurée soit pas spectrophotométrie UV visible, soit par chromatographie liquide haute performance à phase inversée.

On note que, quelles que soient les concentrations en ciclosporine, la teneur en alcool résiduel, en l'espèce en éthanol, est faible, à savoir inférieure à 12 mg/ml. Par ailleurs la teneur en éthanol résiduel est contrôlée. En effet, la solution huileuse de ciclosporine utilisée comme matière première, soit prête à l'emploi soit préparée spécifiquement, contient de l'huile de ricin polyoxyéthylénée et de l'éthanol.

En d'autres termes, il s'agit d'une solution de cyclosporine dans de l'huile hydro-dispersive et émulsifiante, par exemple de l'huile de ricin polyoxyéthylénée, sans autre huile, sans autres agents, donc sans polysorbate ou tensio-actif. Seul de l'éthanol est présent. La concentration en éthanol de la solution huileuse de ciclosporine utilisée comme matière première peut atteindre 278 mg/ml.

La présence d'éthanol permet d'améliorer la solubilité de la ciclosporine dans la phase huileuse mais est défavorable pour la tolérance oculaire, en raison de propriétés pro-inflammatoire et irritante pour la cornée. Il doit donc être retiré après solubilisation de la ciclosporine dans l'huile de ricin polyoxyéthylénée afin d'obtenir un collyre ayant une bonne tolérance à l'instillation.

### Exemple 2 :

Le tableau suivant donne les formulations détaillées d'un autre mode de préparation pour des concentrations en ciclosporine de 1, 10 et 20 mg/ml dans lequel l'agent lubrifiant et cicatrisant est à base de povidone.

La povidone est un lubrifiant oculaire. C'est un polymère synthétique contenant des chaînes linéaires de 1-vinyl-2-pyrrolidone. Elle supplée temporairement à l'insuffisance de larmes. Avantageusement, elle est utilisée sous forme de solution prête à l'emploi et exempte de conservateur ou sous forme de poudre diluée extemporanément dans de l'eau stérile apyrogène (eau PPI) ou BSS Balanced Saline Solution (solution pour irrigation oculaire)
L'huile préférentiellement utilisée est comme pour le premier exemple, de l'huile de ricin polyoxyéthylénée.

Le tableau 2 suivant donne les formulations détaillées d'une telle préparation pour des concentrations en ciclosporine de 1, 10 et 20 mg/ml.

**Tableau 2**

| | Formulation 2 | | |
|---|---|---|---|
| Ciclosporine | 1 mg/ml | 10 mg/ml | 20 mg/ml |
| Huile de ricin polyoxyéthylénée | 13 mg/ml | 130 mg/ml | 260 mg/ml |
| Ethanol | 0-5,56 mg/ml | 0 - 5,56 mg/ml | 0 - 11,12 mg/ml |
| Azote | Traces éventuelles | Traces éventuelles | Traces éventuelles |
| Solution aqueuse (eau PPI) | QSP | QSP | QSP |
| dont : povidone | 14,7 mg/ml | 13,125 mg/ml | 11,25 mg/ml |
| dont : chlorure de sodium, acétate de sodium, citrate de sodium, chlorure de calcium, chlorure de magnésium hexahydrate, chlorure de potassium, hydroxyde de sodium, acide chlorhydrique | | | |
| % de phase huileuse | 2% | 12,50% | 25% |
| % de phase aqueuse | 98% | 87,50% | 75% |

Une variation de plus ou moins 10% des valeurs de concentration cible indiquées est acceptable.

La concentration en ciclosporine A est mesurée soit pas spectrophotométrie UV visible, soit par chromatographie liquide haute performance à phase inversée.

On note que, ici aussi et de façon similaire à l'exemple 1, quelles que soient les concentrations en ciclosporine, la teneur en alcool résiduel, en l'espèce en éthanol, est faible, à savoir inférieure à 12 mg/ml et contrôlée.

Dans les deux exemples on note que la proportion d'huile dans l'émulsion varie de 2% à 25%. Ces proportions sont celles nécessaires à la solubilisation et à la stabilité de l'émulsion ou de la microémulsion car elles permettent d'éviter la précipitation de la ciclosporine A.

La préparation de ces formulations de collyre est maintenant donnée dans le cas d'un collyre contenant de l'acide hyaluronique, étant entendu que la préparation est identique lorsque le collyre comprend de la povidone.

La première étape consiste à éliminer, et au moins à 99% en masse de l'alcool présent dans la solution huileuse de ciclosporine, en l'espèce l'éthanol. Cette élimination est, par exemple, réalisée dans les conditions suivantes :
À une température maîtrisée, et qui est au maximum de 45°C, et avantageusement voisine de 25°C, un flux d'azote sous une pression de 0,5 bar est dirigé et concentré par l'intermédiaire d'une aiguille, sur la surface de la solution huileuse à traiter. Le traitement est appliqué durant la durée nécessaire, étant entendu que cette durée est au minimum de 14 heures.

L'azote est un gaz neutre qui ne provoque pas d'altérations chimiques des autres composants de la formulation.

Il est également possible d'éliminer l'éthanol par d'autres techniques connues en soi, par exemple par évaporation passive à l'air libre ou sous vide.

Néanmoins, l'utilisation d'un flux d'azote est une technique d'évaporation préférée car elle est facile à mettre en œuvre et à contrôler, tout en étant plus rapide que l'évaporation passive. A titre d'exemple, la concentration finale en éthanol est inférieure à 11,12 mg/ml lorsque l'on prépare un collyre dont la concentration en ciclosporine est de 20mg/ml.

L'étape suivante consiste à diluer, en solution aqueuse, le concentré de ciclosporine en solution huileuse obtenu à l'étape précédente. Avantageusement, lors de cette étape, on introduit une solution aqueuse de lubrifiant/cicatrisant oculaire. Ici il s'agit d'un mélange d'acide hyaluronique et d'hyaluronate de sodium. La solution aqueuse contenant le lubrifiant/cicatrisant est soit prête à l'emploi, soit préparée extemporanément. Dans ce cas, la solution est avantageusement préparée à partir d'une poudre du lubrifiant/cicatrisant souhaité et d'eau PPI. Dans tous les cas, la conformité de la solution est contrôlée par la PUI.

L'incorporation est avantageusement réalisée à température ambiante, avantageusement inférieure à 25°C, et dans des conditions aseptiques ou au moins dites propres.

L'étape suivante consiste à incorporer la phase aqueuse ainsi obtenue et à réaliser l'émulsion, la microémulsion ou la solution micellaire. Pour cela, on effectue le mélange de la solution aqueuse obtenue à l'étape précédente avec le concentré de ciclosporine A en solution huileuse disponible à l'issue de la première étape de concentration. Le mélange entre les deux phases lipophile et hydrophile est généré par apport d'énergie cinétique, en agitant la solution, par exemple à l'aide d'un agitateur à barreau magnétique ou par une autre technique connue en soi. Ainsi, la totalité de la fraction huileuse contenant la ciclosporine est dispersée dans la phase aqueuse sous agitation afin d'obtenir une émulsion, microémulsion ou solution micellaire, étant entendu qu'ici la phase aqueuse représente au moins 75% de l'émulsion, microémulsion ou solution micellaire finale.

Le mélange sous agitation est effectué à une température comprise entre 2°C et 45°C, pendant une durée comprise entre 0,05h et 48h. Il est à noter que la solution micellaire obtenue est plus précisément une solution aqueuse micellaire dont la taille de la majorité de micelles colloïdales présentes est inférieure à 25nm.

Une fois la préparation du collyre effectuée, il convient, lors de la dernière étape, de conditionner le collyre en assurant et en conservant la stérilité de la préparation. Une stérilisation terminale est appliquée à la préparation finie au moment du conditionnement dans une zone à atmosphère contrôlée, il s'agit par exemple d'une filtration stérilisante.

Avantageusement, un conditionnement bénéficiant d'une technologie « multidose sans conservateur » préservant la stérilité du collyre après ouverture du contenant ou un conditionnement en emballage unidose stérile est requise du fait de l'absence de conservateur. Ainsi, le collyre est avantageusement présenté sous forme d'un conditionnement identifié, à patient unique, et contenant une concentration définie de ciclosporine, selon la pathologie à traiter.

On conçoit que le collyre peut se présenter sous forme d'un kit comportant plusieurs doses unitaires pour un traitement sur un nombre restreint de jours et/ou un nombre donné de patients. En variante, le collyre est présenté sous la forme d'un flacon multidoses garantissant le maintien de la stérilité après ouverture. Un tel flacon et utilisable pour un nombre de jours définis et validés, par exemple 30 jours.

Des essais ont été réalisés pour contrôler la stabilité de la préparation tout au long de la période de conservation définie, avant et après ouverture, ce qui a permis de définir des conditions de conservation avant et après ouverture.

Le tableau 3 ci-après fournit les données obtenues, lors d'essais de conservation à une température de 4°C et à une température de 25°C. La demanderesse n'a pas constaté de différence significative de stabilité, entre les deux températures de conservation, cela jusqu'à une durée de conservation de 90 jours. Des études complémentaires sont en cours et visent à une extension de la durée de conservation du collyre objet de l'invention.

Ces essais, dans tous les cas, suivent les recommandations en vigueur.

**Tableau 3**

| | | | |
|---|---|---|---|
| Concentration en ciclosporine A | 1 mg/ml | 10 mg/ml | 20 mg/ml |
| Durée de conservation maximale | 90 jours | 90 jours | 90 jours |
| Conservation après ouverture | 30 jours | 30 jours | 30 jours |

D'autre part, à chaque lot de collyre réalisé, la qualité de la préparation finale est vérifiée sur un échantillonnage représentatif du lot produit. Afin de s'assurer que la préparation satisfait aux exigences réglementaires de la Pharmacopée Européenne.

Un étiquetage réglementaire de la préparation est également réalisé, pour identifier les différentes concentrations en ciclosporine et/ou les conditions d'utilisation et/ou de conservation.

En variante, on utilise un signe d'identification visuel des différentes concentrations en ciclosporine.

On conçoit qu'une telle préparation trouve son application non seulement pour un usage humain mais également vétérinaire.

## Revendications

1. Procédé de préparation d'un collyre de ciclosporine A, dont la concentration dans le collyre est comprise entre 1 mg/ml et 40 mg/ml, sous forme d'une émulsion, d'une microémulsion ou d'une solution micellaire lipophile/hydrophile dont la phase lipophile, donc huileuse, contient la ciclosporine A,
**caractérisé en ce qu'**il comprend au moins des étapes consistant à :
a) éliminer au moins 99% en masse de l'éthanol contenu dans la solution huileuse de ciclosporine A, sous un flux d'azote sous une pression de 0,5 bar, à une température comprise entre 2°C et 45°C et pendant une durée comprise entre 0,25h et 24h,
b) diluer en solution aqueuse le concentré obtenu à l'étape a),
c) réaliser une émulsion, une microémulsion ou une solution micellaire dont la phase aqueuse représente au moins 75% de l'émulsion, microémulsion ou solution micellaire finale, par mélange sous agitation de la solution huileuse concentrée obtenue à l'étape a) avec la solution aqueuse diluée obtenue à l'étape b),
d) conditionner stérilement le collyre obtenu dans des conditionnements adaptés à un usage ophtalmique.

2. Procédé selon la revendication 1, **caractérisé en ce que** le collyre est préparé sans ajout de conservateur antimicrobien.

3. Procédé selon la revendication 1 ou 2, **caractérisé en ce que** lors de l'étape b), on incorpore à la solution aqueuse une solution d'agent lubrifiant et/ou cicatrisant.

4. Procédé selon la revendication 3, **caractérisé en ce que** l'agent lubrifiant et/ou cicatrisant est choisi parmi l'acide hyaluronique et ses sels ou la povidone.

5. Procédé selon la revendication 3 ou 4, **caractérisé en ce que** la solution d'agent lubrifiant et/ou cicatrisant est reconstituée à partir de poudre ou prête à l'emploi.

6. Procédé selon l'une des revendications précédentes, **caractérisé en ce que** lors de l'étape d), le collyre est soumis à une stérilisation finale préalablement à son conditionnement.

7. Procédé selon la revendication 1, **caractérisé en ce que** la solution huileuse de ciclosporine A est une solution de ciclosporine A dans de l'huile hydro-dispersive et émulsifiante.

8. Procédé selon la revendication 7, **caractérisé en ce que** la solution huileuse de ciclosporine A est une solution de ciclosporine A dans de l'huile de ricin polyoxyéthylénée.

9. Procédé selon la revendication 1, **caractérisé en ce que** la concentration en cyclosporine A dans le collyre obtenu à l'issue de l'étape d) est comprise entre 5 mg/ml et 25 mg/ml.

10. Procédé selon l'une des revendications précédentes, **caractérisé en ce que** le collyre obtenu à l'issue de l'étape d) est adapté à un usage humain ou vétérinaire.

## Patentansprüche

1. Verfahren zur Herstellung von Augentropfen aus Cyclosporin A, dessen Konzentration in den Augentropfen zwischen 1 mg/ml und 40 mg/ml liegt, in Form einer Emulsion, einer Mikroemulsion oder einer lipophilen/hydrophilen mizellaren Lösung, deren lipophile, somit ölige Phase, Cyclosporin A enthält,
**dadurch gekennzeichnet, dass** es mindestens Schritte umfasst, bestehend aus:
a) Entfernen von mindestens 99 Gew.-% des in der öligen Lösung von Cyclosporin A enthaltenen Ethanols, unter einem Stickstoffstrom bei einem Druck von 0,5 bar, bei einer Temperatur zwischen 2 °C und 45 °C, über eine Zeitdauer zwischen 0,25 h und 24 h,
b) Verdünnen des in Schritt a) erhaltenen Konzentrats in wässriger Lösung,
c) Herstellen einer Emulsion, einer Mikroemulsion oder einer mizellaren Lösung, deren wässrige Phase mindestens 75 % der endgültigen Emulsion, Mikroemulsion oder mizellaren Lösung darstellt, durch Mischen unter Rühren der in Schritt a) erhaltenen konzentrierten öligen Lösung mit der in Schritt b) erhaltenen verdünnten wässrigen Lösung,
d) Steriles Konditionieren der Augentropfen in für einen ophthalmischen Gebrauch angepassten Behältern.

2. Verfahren nach Anspruch 1, **dadurch gekennzeichnet, dass** die Augentropfen ohne Zusatz eines antimikrobiellen Konservierungsmittels hergestellt werden.

3. Verfahren nach Anspruch 1 oder 2, **dadurch gekennzeichnet, dass** in Schritt b) eine Gleit- und/oder Wundheilmittellösung in die wässrige Lösung integriert wird.

4. Verfahren nach Anspruch 3, **dadurch gekennzeichnet, dass** das Gleit- und/oder Wundheilmittel ausgewählt ist aus Hyaluronsäure und ihren Salzen oder Povidon.

5. Verfahren nach Anspruch 3 oder 4, **dadurch gekennzeichnet, dass** die Gleit- und/oder Wundheilmittellösung aus Pulver rekonstituiert wird oder gebrauchsfertig ist.

6. Verfahren nach einem der vorhergehenden Ansprüche, **dadurch gekennzeichnet, dass** in Schritt d) die Augentropfen vor ihrem Konditionieren einer abschließenden Sterilisation unterzogen werden.

7. Verfahren nach Anspruch 1, **dadurch gekennzeichnet, dass** die ölige Lösung von Cyclosporin A eine Lösung von Cyclosporin A in wasserdispergierendem und emulgierendem Öl ist.

8. Verfahren nach Anspruch 7, **dadurch gekennzeichnet, dass** die ölige Lösung von Cyclosporin A eine Lösung von Cyclosporin A in Polyoxyethylen-Rizinusöl ist.

9. Verfahren nach Anspruch 1, **dadurch gekennzeichnet, dass** die Konzentration von Cyclosporin A in den aus Schritt d) erhaltenen Augentropfen zwischen 5 mg/ml und 25 mg/ml liegt.

10. Verfahren nach einem der vorhergehenden Ansprüche, **dadurch gekennzeichnet, dass** die nach Schritt d) erhaltenen Augentropfen für die human- oder tierärztliche Verwendung angepasst sind.

## Claims

1. A method for preparing eye drops of cyclosporin A, the concentration of which in the eye drops is between 1 mg/ml and 40 mg/ml, in the form of an emulsion, a microemulsion or a lipophilic/hydrophilic micellar solution of which the lipophilic, and therefore oily, phase contains cyclosporin A,
**characterised in that** the method comprises at least the steps of:
a) eliminating at least 99 wt.% of the ethanol contained in the oily solution of cyclosporin A, under a flow of nitrogen under a pressure of 0.5 bar, at a temperature between 2°C and 45°C and for a period between 0.25 h and 24 h,
b) diluting the concentrate obtained in step a) in an aqueous solution,
c) forming an emulsion, a microemulsion or a micellar solution of which the aqueous phase makes up at least 75% of the final emulsion, microemulsion or micellar solution, by mixing, under stirring, the concentrated oily solution obtained in step a) with the diluted aqueous solution obtained in step b),
d) packaging, in a sterile manner, the obtained eye drops in packaging that is suitable for ophthalmic use.

2. The method according to claim 1, **characterised in that** the eye drops are prepared without the addition of antimicrobial preservatives.

3. The method according to claim 1 or 2, **characterised in that** a solution of lubricating and/or wound-healing agent is incorporated into the aqueous solution during step b).

4. The method according to claim 3, **characterised in that** the lubricating and/or wound-healing agent is selected from hyaluronic acid and the salts thereof or povidone.

5. The method according to claim 3 or 4, **characterised in that** the solution of lubricating and/or wound-healing agent is reconstituted from powder or is ready to use.

6. The method according to any one of the preceding claims, **characterised in that** during step d) the eye drops are subjected to final sterilisation prior to being packaged.

7. The method according to claim 1, **characterised in that** the oily solution of cyclosporin A is a solution of cyclosporin A in hydrodispersive and emulsifying oil.

8. The method according to claim 7, **characterised in that** the oily solution of cyclosporin A is a solution of cyclosporin A in polyoxyethylated castor oil.

9. The method according to claim 1, **characterised in that** the concentration of cyclosporin A in the eye drops obtained at the end of step d) is between 5 mg/ml and 25 mg/ml.

10. The method according to any one of the preceding claims, **characterised in that** the eye drops obtained at the end of step d) are suitable for human or veterinary use.
